(19) Europäisches Patentamt
European Patent Office
Office européen des brevets

(11) EP 4 265 657 A1

(12) **EUROPEAN PATENT APPLICATION**
published in accordance with Art. 153(4) EPC

(43) Date of publication:
**25.10.2023 Bulletin 2023/43**

(21) Application number: **21910479.1**

(22) Date of filing: **14.12.2021**

(51) International Patent Classification (IPC):
***C08F 220/10*** *(2006.01)* ***C08F 230/08*** *(2006.01)*
***C08K 5/13*** *(2006.01)* ***C08L 101/10*** *(2006.01)*
***A61K 8/00*** *(2006.01)* ***A61K 8/89*** *(2006.01)*

(52) Cooperative Patent Classification (CPC):
**A61K 8/00; A61K 8/89; C08F 220/10; C08F 230/08; C08K 5/13; C08L 101/10**

(86) International application number:
**PCT/JP2021/046062**

(87) International publication number:
**WO 2022/138330 (30.06.2022 Gazette 2022/26)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **21.12.2020 JP 2020211246**

(71) Applicant: **Dow Toray Co., Ltd.**
**Tokyo 140-8617 (JP)**

(72) Inventors:
- **AKIYAMA, Katsuhiro**
  **Ichihara-shi Chiba 299-0108 (JP)**
- **SOUDA, Tatsuo**
  **Ichihara-shi Chiba 299-0108 (JP)**
- **SUGIURA, Tsunehito**
  **Ichihara-shi Chiba 299-0108 (JP)**

(74) Representative: **Murgitroyd & Company**
**Murgitroyd House**
**165-169 Scotland Street**
**Glasgow G5 8PL (GB)**

(54) **RADICAL COPOLYMER COMPOSITION, PRODUCTION METHOD THEREFOR, AND COSMETIC OR COSMETIC INGREDIENT CONTAINING RADICAL COPOLYMER COMPOSITION**

(57) [Problem]

To provide a monomer composition which has no concern about safety when used as a raw material of a cosmetic material or the like and does not cause deterioration in quality such as gelation, and a manufacturing method for the monomer composition.

[Resolution Means]

A radical copolymer composition, comprising a radical copolymer of:
a) a radical polymerizable monomer having a radical polymerizable organic group and an organic silicon-containing organic group in a molecule; and
b) a radical polymerizable monomer other than component a);

wherein the total concentration of a polymerization inhibitor based on the total mass of the radical copolymer and the polymerization inhibitor is 50 ppm by mass or less; and a manufacturing method thereof.

EP 4 265 657 A1

## Description

### TECHNICAL FIELD

The present invention relates to a radical copolymer composition, a method of manufacturing the radical copolymer composition, and a cosmetic material or a cosmetic raw material containing the radical copolymer composition.

### BACKGROUND ART

Use of a polymer prepared from a monomer composition containing a radical polymerizable group as a film-forming agent is known, in order to improve the water resistance and sebum resistance of cosmetic materials, in particular make-up cosmetic materials, thereby improving the cosmetic durability. In particular, an organopolysiloxane containing a radical polymerizable organic group can impart water repellency and slipperiness to a cosmetic material. However, there are cases where there is a problem with compatibility to cosmetic raw materials to be added and the like.

In order to solve such problems, copolymers having a carbosiloxane dendrimer structure or a siloxane macromonomer structure have been proposed. For example, Patent Document 1 proposes a copolymer based on an unsaturated monomer having a specific carbosiloxane dendrimer structure, and shows that the copolymer can be used as a film-forming agent which is excellent in blending stability with cosmetic raw materials such as an ultraviolet absorber or the like, and also excellent in water resistance and sebum resistance.

On the other hand, in order to manufacture a monomer having a complex structure such as a carbosiloxane dendrimer structure, a siloxane macromonomer structure, and the like, it is necessary to go through a plurality of steps. In particular, some raw materials in the upstream process have high reactivity. Therefore, measures are generally taken to prevent the raw materials from reacting and gelling during storage of the raw materials and during the process by adding a polymerization inhibitor, and the like.

For example, Patent Document 2 describes a silicone monomer composition including a silicone monomer containing a polymerizable group and 5 to 400 ppm of a polymerization inhibitor having a specific structure with respect to the silicone monomer. Furthermore, Patent Document 3 describes that the cosmetic composition contains a 10 ppm to 20 mass% of a polymerization inhibitor with respect to the total mass of the composition in order to increase the stability of the cosmetic composition over time.

However, it has been pointed out that some polymerization inhibitors are suspected to be mutagenic or the like, and when used in direct contact with the human body as with cosmetic materials, the content is preferably reduced as much as possible from the perspective of safety. Therefore, it is desirable to separate the polymerization inhibitor from the monomer composition, but it is difficult to remove the polymerization inhibitor by means such as distillation, particularly if the raw material is an organopolysiloxane containing a radical polymerizable organic group, or the like. Specifically, although it is necessary to heat the monomer composition for distillation, there is a problem in that unintended polymerization and gelation may occur and the amount of the polymerization inhibitor cannot be reduced to a certain amount or lower.

### Prior Art Documents

### Patent Documents

Patent Document 1: Japanese Unexamined Patent Application 2014-40512
Patent Document 2: Japanese Unexamined Patent Application 2004-115790
Patent Document 3: Japanese Unexamined Patent Application 2006-169234

### SUMMARY OF THE INVENTION

### PROBLEM TO BE SOLVED BY THE INVENTION

An object of the present invention is to solve the aforementioned problems of the conventional technology, provide a radical copolymer composition which has no concern about safety when used as a raw material of a cosmetic material or the like and that does not cause deterioration of quality such as gelation, and to provide a method manufacturing the same.

## MEANS FOR SOLVING THE PROBLEM

As a result of conducting diligent research on the problem described above, the present inventors arrived at the present invention. In other words, an object of the present invention is achieved by:

a radical copolymer composition, containing a radical copolymer of:

a) a radical polymerizable monomer having a radical polymerizable organic group and an organic silicon-containing organic group in a molecule; and
b) a radical polymerizable monomer other than component a);

wherein the total concentration of a polymerization inhibitor based on the total mass of the radical copolymer and the polymerization inhibitor is 50 ppm by mass or less.

The present invention also relates to a method of manufacturing the monomer radical copolymer composition of the present invention, including: a step of bringing a liquid containing a radical polymerizable monomer into contact with an adsorbing agent selected from a group consisting of activated carbon and alumina. The invention also relates to a cosmetic material or cosmetic raw material containing the radical copolymer composition of the present invention.

## EFFECTS OF THE INVENTION

The amount of the polymerization inhibitor is reduced to an extremely low level, and thus there is no concern about safety even when the radical copolymer composition of the present invention is used as a raw material for applications in direct contact with the human body such as cosmetic materials and the like, or in raw materials thereof.
In addition, since the manufacturing method for a radical copolymer composition of the present invention does not include a heating step by distillation or the like, even though the amount of the polymerization inhibitor is low, gelation or the like due to an unintended reaction does not occur, and a high-quality radical copolymer composition can be provided.

## BRIEF DESCRIPTION OF THE DRAWINGS

FIG. 1 schematically depicts a device for separating a polymerization inhibitor in a method of manufacturing a radical copolymer composition of the Examples.

## MODE FOR CARRYING OUT THE INVENTION

[Radical Polymerizable Monomer Having Radical Polymerizable Organic Group and Organic Silicon-Containing Organic Group in Molecule]
The radical copolymer composition of the present invention contains a radical polymerizable monomer having a radical polymerizable organic group and an organic silicon-containing organic group in a molecule as a component constituting the radical copolymer ("component a)").
Examples of the organic silicon-containing organic group included in the radical polymerizable monomer include a carbosiloxane dendrimer structure and a branched or linear siloxane structure. A carbosiloxane dendrimer structure is preferred.
The carbosiloxane dendrimer structure is a chemical structure that is highly branched radially from one silicon atom, and the radical polymerizable monomer having the carbosiloxane dendrimer structure is preferably a monomer expressed by the following general formula (1):

[Formula 1]

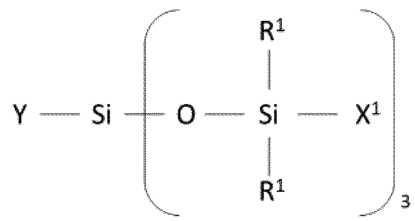

(1)

{where

Y is a radical polymerizable organic group,
$R^1$ is an alkyl group, an aryl group or a trimethylsiloxy group;
$X^1$ is a silylalkyl group expressed by the following general formula (2) when i = 1:

[Formula 2]

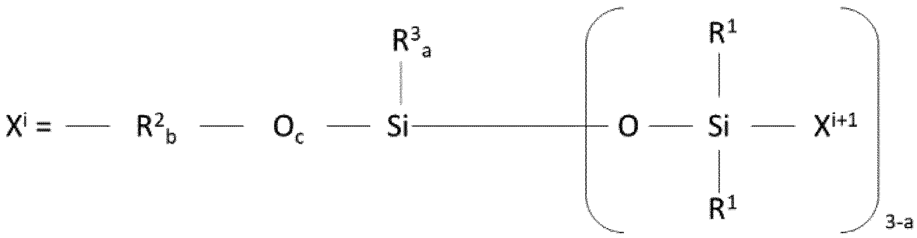

(2)

(where
$R^1$ is the same group as defined for general formula (1);
$R^2$ is an alkylene group with 2 to 10 carbon atoms;
$R^3$ is a group selected from a group consisting of alkoxy groups, hydroxyl groups, alkyl groups, aryl groups, and trimethylsiloxy groups;
$X^{i+1}$ is a group selected from a group consisting of hydrogen atoms, alkyl groups with 1 to 10 carbon atoms, aryl groups, and the above silylalkyl groups, where i is an integer from 1 to 10, indicating the hierarchy of the silylalkyl group; a is an integer from 0 to 3; and
(b and c are 0 or 1.)}

The radical polymerizable organic group is not particularly limited so long as the organic group can be radically reacted, and specific examples include (meth)acryloxy group-containing organic groups, (meth)acrylamide group-containing organic groups, and styryl group-containing organic groups, and alkenyl groups with 2 to 10 carbon atoms. An organic group containing a (meth)acryloyl group is preferable. Examples of the radical polymerizable organic group include organic groups expressed by the following general formula.

[Formula 3]

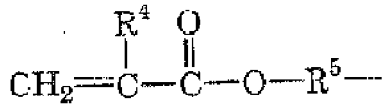

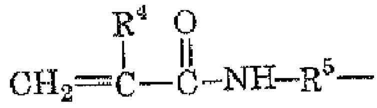

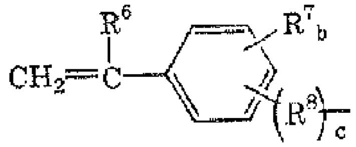

(where $R^4$ and $R^6$ are hydrogen atoms or methyl groups, $R^5$ and $R^8$ are alkylene groups with 1 to 10 carbon atoms, and $R^7$ is an alkyl group with 1 to 10 carbon atoms. b is an integer from 0 to 4, and c is 0 or 1).

Examples of such radical polymerizable organic groups include acryloxymethyl groups, 3-acryloxypropyl groups, methacryloxymethyl groups, 3-methacryloxypropyl groups, 4-vinylphenyl groups, 3-vinylphenyl groups, 4-(2-propenyl) phenyl groups, 3-(2-propenyl) phenyl groups, 2-(4-vinylphenyl)ethyl groups, 2-(3-vinylphenyl)ethyl groups, vinyl groups, allyl groups, methallyl groups, and 5-hexenyl groups.

In the general formulas (1) and (2), $R^1$ is an alkyl group, an aryl group or a trimethylsiloxy group, preferably a methyl group or a phenyl group, and of these, a methyl group is particularly preferable.

In general formula (2), $R^2$ is an alkylene group with 2 to 10 carbon atoms, and ethylene groups, methylethylene groups, hexylene groups, 1-methylpentylene groups, and of these, 1,4-dimethylbutylene groups are preferable.

In the general formula (2), $R^3$ is a group selected from a group consisting of an alkoxy group, a hydroxyl group, an alkyl group, an aryl group, and a trimethylsiloxy group. The alkyl group is preferably an alkyl group with 1 to 10 carbon atoms, and examples thereof include methyl groups, ethyl groups, propyl groups, butyl groups, and isopropyl groups.

In general formula (2), $X^{i+1}$ is a hydrogen atom or a group selected from a group consisting of alkyl groups with 1 to 10 carbon atoms, aryl groups, and the abovementioned silylalkyl groups. i is an integer from 1 to 10, and indicates the hierarchical number of the silylalkyl group, or in other words, the number of repetitions of the silylalkyl group. Thus, when the hierarchical number is 1, the radical polymerizable monomer is expressed by the following general formula.

[Formula 4]

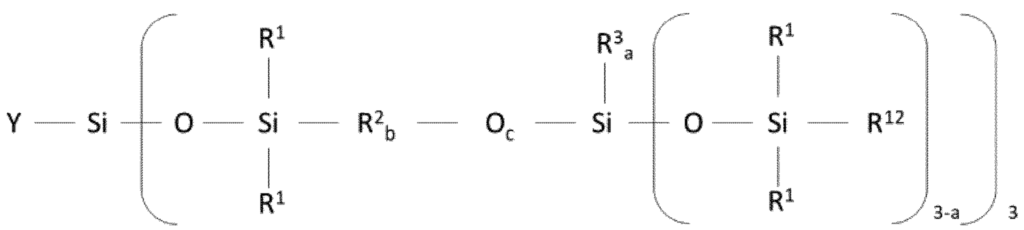

(where Y, $R^1$, $R^2$ and $R^3$ are the same groups as defined for the general formulas (1) and (2), and $R^{12}$ is a hydrogen atom or the same groups as $R^1$ above, a is the same number as defined for general formula (1), but the average total number of a in one molecule is 0 to 7.) When the hierarchical number is 2, the radical polymerizable monomer is expressed by the following general formula.

[Formula 5]

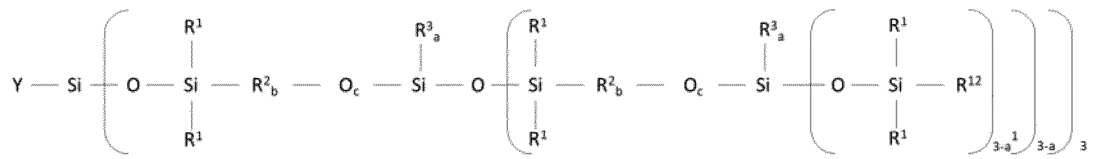

(where Y, $R^1$, $R^2$, $R^3$ and $R^{12}$ are the same groups as defined for general formulas (1) and (2). a and $a^1$ are the same numbers as defined for general formula (1), but the average total number of a and a' in one molecule is 0 to 25.)

Examples of the radical polymerizable monomer having the carbosiloxane dendrimer structure of this component include the monomers expressed by the following average compositional formula.

[Formula 6]

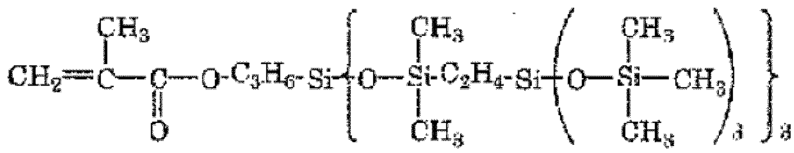

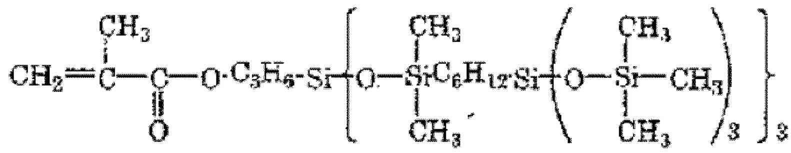

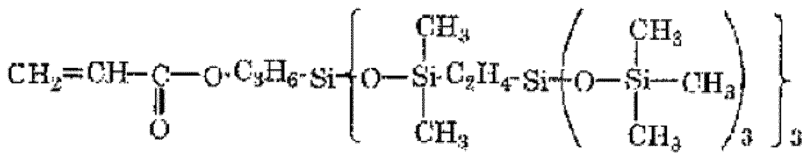

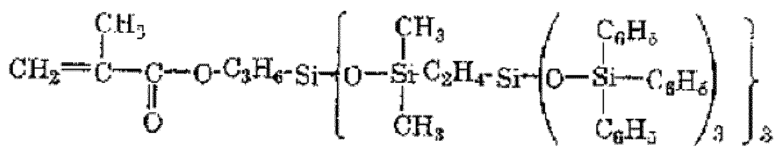

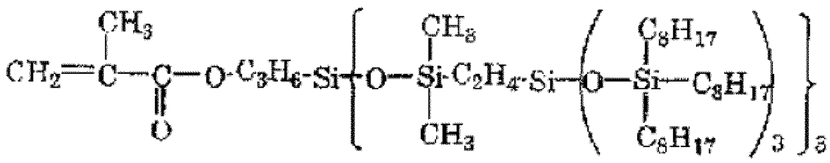

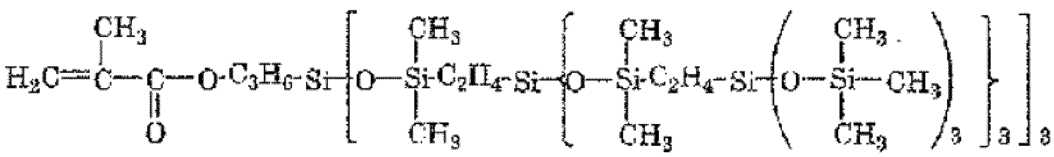

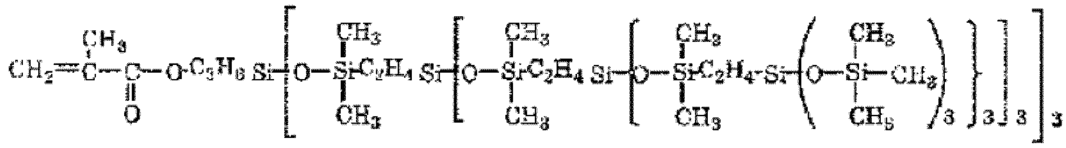

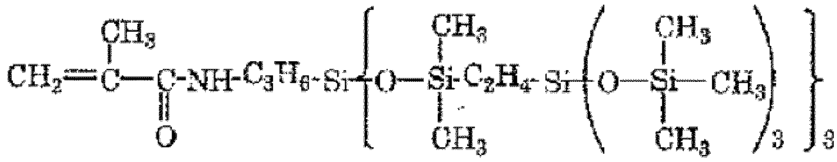

[Formula 7]

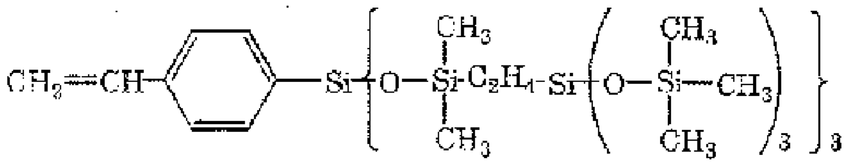

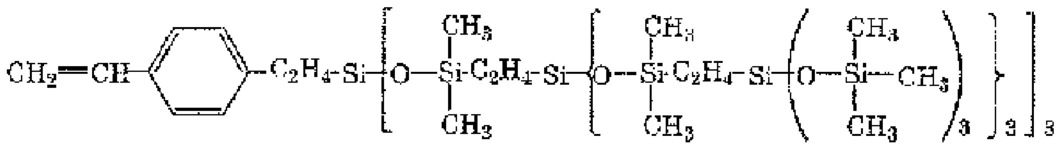

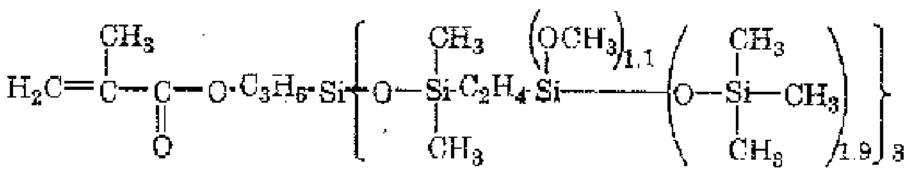

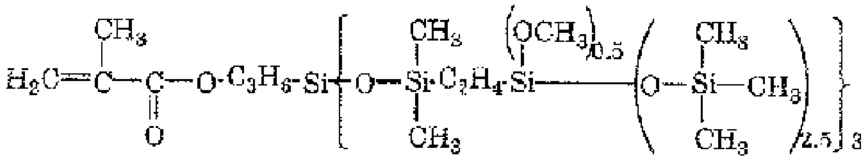

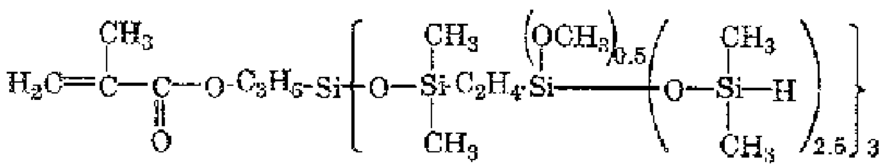

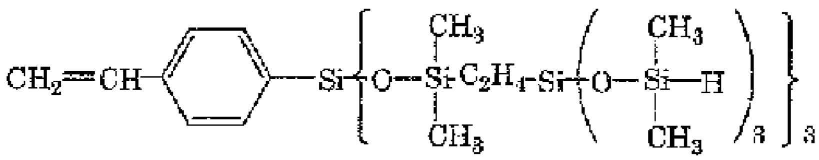

Such carbosiloxane dendrimers can be manufactured in accordance with a method of manufacturing a branched siloxane-silalkylene copolymer described in Japanese Unexamined Patent Application H11-1530. For example, the monomer can be manufactured by a hydrosilylation reaction between an alkenyl group-containing organic silicon compound with a silicon compound containing a silicon atom-bonded hydrogen atom expressed by the following general formula:

[Formula 8]

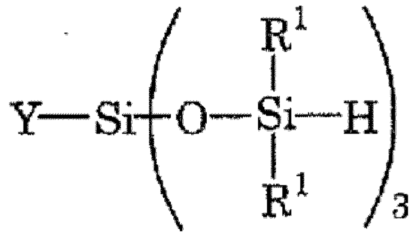

(where, $R^1$ and Y are the same groups as defined for General Formula (1)).

Examples of the silicon compound expressed by the formula above include 3-methacryloxypropyl tris(dimethylsiloxy)silane, 3-acryloxypropyl tris(dimethylsiloxy) silane, and 4-vinylphenyl tris(dimethylsiloxy)silane. As the alkenyl group-containing organic silicon compound, vinyltris(trimethylsiloxy) silane, vinyltris(dimethylphenylsiloxy)silane, and 5-hexenyl tris(trimethylsiloxy) silane can be used. Note that the hydrosilylation reaction is preferably performed in the presence of a transition metal catalyst such as chloroplatinic acid or a platinum vinylsiloxane complex, or the like.

Examples of radical polymerizable monomers with a branched or linear siloxane structure include organopolysiloxanes containing M units expressed by $R^a_3SiO_{1/2}$ or $R^a_2R^bSiO_{1/2}$, D units expressed by $R^a_2SiO_{2/2}$ or $R^aR^bSiO_{2/2}$, T units expressed by $R^aSiO_{3/2}$ or $R^bSiO_{3/2}$, and Q units expressed by $SiO_{4/2}$, at an arbitrary ratio. Herein, $R^a$ is an alkyl group, an aryl group, an aralkyl group, or a group in which some or all of the hydrogen atoms of these groups are substituted with halogen atoms, and $R_b$ is a radical polymerizable organic group. Radical polymerizable monomers with branched or linear siloxane structures are preferably macromonomers with a relatively large weight average molecular weight.

$R^a$ is an alkyl group, an aryl group, an aralkyl group, or a group in which some or all of the hydrogen atoms of these groups are substituted with halogen atoms. An alkyl group with 1 to 10 carbon atoms is preferred as the alkyl group, with examples including methyl groups, ethyl groups, propyl groups, butyl groups, pentyl groups, isopropyl groups, isobutyl groups, cyclopentyl groups, and cyclohexyl groups. Examples of aryl groups include phenyl groups and naphthyl groups. Moreover, examples of aralkyl groups include benzyl groups and phenethyl groups. Examples of groups in which some or all of the hydrogen atoms are substituted with halogen atoms include fluorine-substituted alkyl groups such as trifluoropropyl groups, heptadecafluorodecyl groups, and the like.

$R^b$ is a radical polymerizable organic group, which is not particularly limited, so long as the organic group can be radically reacted, and can be the same groups as defined for the carbosiloxane dendrimer structure. In other words, examples include (meth)acryloxy group-containing organic groups, (meth)acrylamide group-containing organic groups, styryl group-containing organic groups, and alkenyl groups with 2 to 10 carbon atoms.

Radical polymerizable monomers having such a branched or linear siloxane structure can be synthesized using raw materials and methods well known to a person of ordinary skill in the art, and can be obtained, for example, by reacting an organosiloxane having a silanol group in a molecule with an organic chlorosilane compound in the presence of a base. The organic chlorosilane compound is not particularly limited so long as it has a radical polymerizable organic group, and examples include 3-methacryloxypropyl dimethylchlorosilane and 3-methacryloxypropyl dichloromethylsilane.

[Radical Polymerizable Monomer Other Than Component a)]

The radical copolymer composition of the present invention contains a radical polymerizable monomer other than component a) as a component constituting the radical copolymer ("component b)"). In other words, the radical copolymer included in the radical copolymer composition of the present invention is a radical copolymer of component a) and component b).

Component b) is not particularly limited provided that copolymerizing with component a) is possible, and examples thereof include monomers containing an unsaturated bond such as a monovalent hydrocarbon group having a carbon-carbon double bond at a molecular chain terminal or a group derived from a monovalent unsaturated carboxylic acid, such as vinyl groups, allyl groups, (meth)acryl groups, (meth) acryloxy groups, and the like. Component b) is preferably an acrylic ester monomer or methacrylic ester monomer with 4 to 13 carbon atoms. In particular, an acrylic acid ester or methacrylic acid ester with 4 to 10 carbon atoms is more preferable.

Specifically, examples of component b) include: lower alkyl (meth)acrylates such as methyl (meth)acrylate,

ethyl (meth)acrylate, n-propyl (meth)acrylate, isopropyl (meth)acrylate, and the like; glycidyl (meth)acrylate; higher (meth)acrylates such as n-butyl (meth)acrylate, isobutyl (meth)acrylate, tert-butyl (meth)acrylate, n-hexyl (meth)acrylate, cyclohexyl (meth)acrylate, 2-ethylhexyl (meth)acrylate, octyl (meth)acrylate, lauryl (meth)acrylate, stearyl (meth)acrylate, isostearyl (meth)acrylate, behenyl (meth)acrylate, and the like; lower fatty acid vinyl esters such as vinyl acetate, vinyl propionate, and the like; higher fatty acid esters such as vinyl butyrate, vinyl caproate, vinyl 2-ethylhexanoate, vinyl laurate, vinyl stearate, and the like; aromatic vinyl monomers such as styrene, vinyl toluene, benzyl (meth)acrylate, phenoxyethyl (meth)acrylate, vinyl pyrrolidone, and the like; amide group-containing vinyl monomers such as (meth)acrylamide, N-methylol (meth)acrylamide, N-methoxymethyl (meth)acrylamide, isobutoxymethoxy (meth)acrylamide, N,N-dimethyl (meth)acrylamide, and the like; hydroxyl group-containing vinyl monomers such as hydroxyethyl (meth)acrylate, hydroxypropyl alcohol (meth)acrylate, and the like; ether bond-containing vinyl monomers such as tetrahydrofurfuryl (meth)acrylate, butoxyethyl (meth)acrylate, ethoxydiethylene glycol (meth)acrylate, polyethylene glycol (meth)acrylate, polypropylene glycol mono(meth)acrylate, hydroxybutyl vinyl ether, cetyl vinyl ether, 2-ethylhexyl vinyl ether, and the like; unsaturated group-containing silicone compounds such as (meth)acryloxypropyl trimethoxysilane, polydimethyl siloxanes containing a (meth)acrylic group at one end, both ends and/or a side chain, a polydimethyl siloxane containing a styryl group at one end, and the like; butadiene; vinyl chloride; vinylidene chloride; (meth)acrylonitrile; dibutyl fumarate; maleic anhydride; dodecyl succinic anhydride; (meth)acrylic glycidyl ether; quaternary ammonium salts derived from (meth)acrylic acids such as 2-hydroxy-3-methacryloxy propyl trimethylammonium chloride and the like; methacrylates of alcohols having a tertiary amine group such as diethylamime methacrylate and the like; and quaternary ammonium salts thereof. The radical polymerizable monomer of component b) may be one type or may be a combination of two or more types.

Furthermore, a polyfunctional vinyl-based monomer can also be used, and examples include trimethylolpropane tri(meth)acrylate, pentaerythritol tri(meth)acrylate, ethylene glycol di(meth)acrylate, tetraethylene glycol di(meth)acrylate, polyethylene glycol di(meth)acrylate, 1,4-butanediol di(meth)acrylate, 1,6-hexanediol di(meth)acrylate, neopentyl glycol di(meth)acrylate, trimethylolpropane trioxyethyl (meth)acrylate, tris(2-hydroxyethyl)isocyanurate di(meth)acrylate, tris(2-hydroxyethyl)isocyanurate tri(meth)acrylate, styryl group-capped polydimethyl siloxane, other such unsaturated group-containing silicone compounds, and the like.

The copolymer included in the radical copolymer composition of the present invention has an acid value of 5 to 300 mgKOH/g, and preferably 35 to 100 mgKOH/g, when measured in accordance with JIS1557-5 with regard to the form prior to neutralization. Furthermore, from the perspective of ease of blending into the cosmetic material, the number average molecular weight of the copolymer is preferably 2000 to 200,000, and more preferably 3000 to 80,000. Furthermore, examples of the nature thereof include liquid, rubber, paste, solid, and powder.

[Method of Manufacturing Radical Copolymer]

The copolymer included in the radical copolymer composition of the present invention can be manufactured by polymerizing a monomer composition containing components a) and b). The manufacturing method has a step (first step) of adding a polymerization initiator to the monomer composition, and carrying out a polymerization reaction. Thereafter, the manufacturing method may have a step (second step) of optionally contacting the obtained polymerization reaction product with a palladium catalyst.

First Step

A radical polymerization method or an ion polymerization method is used as the polymerization method that is used in the polymerization reaction carried out in the first step, and a radical polymerization method is preferable. With regard to the radical polymerization method, a solution polymerization method is preferably used. The solution polymerization is performed by reacting the monomer composition containing component a) and component b) in a solvent under a temperature condition of 50 to 150°C for 3 to 20 hours in the presence of a radical initiator. Examples of the solvent used in the polymerization reaction include aliphatic hydrocarbons such as hexane, octane, decane, cyclohexane, and the like; aromatic hydrocarbons such as benzene, toluene, xylene, and the like; ethers such as diethyl ether, dibutyl ether, tetrahydrofuran, dioxane, and the like; ketones such as acetone, methyl ethyl ketone, methyl isobutyl ketone, diisobutyl ketone, and the like; esters such as methyl acetate, ethyl acetate, butyl acetate, isobutyl acetate, and the like; alcohols such as methanol, ethanol, isopropyl alcohol, butanol, and the like; and organosiloxane oligomers such as octamethylcyclotetrasiloxane, decamethylcyclopentasiloxane, hexamethyldisiloxane, octamethyltrisiloxane, and the like.

Conventionally known compounds commonly used in radical polymerization methods are used as the radical initiator, and specific examples include azobis-based compounds such as 2,2'-azobis(isobutyronitrile), 2,2'-azobis(2-methylbutyronitrile), 2,2'-azobis(2,4-dimethylvaleronitrile), and the like; and organic peroxides such as benzoyl peroxide, lauroyl peroxide, tert-butylperoxy benzoate, tert-butylperoxy-2-ethylhexanoate, tert-hexylperoxy-2-ethylhexanoate, and the like. One type of this radical initiator may be used alone, or two or more types may be mixed and used. The amount

of the radical initiator used is preferably in a range of 0.1 to 5 parts by mass with regard to a total of 100 parts by mass of the monomer composition.

In addition, a chain transfer agent can be added during polymerization. Specific examples of the chain transfer agent include mercapto compounds such as 2-mercaptoethanol, butyl mercaptan, n-dodecyl mercaptan, 3-mercaptopropyl trimethoxysilane, polydimethylsiloxanes having a mercaptopropyl group, and the like; and halides such as methylene chloride, chloroform, carbon tetrachloride, butyl bromide, 3-chloropropyl trimethoxysilane, and the like.

Second Step

The polymerization reaction product obtained in the first step may be contacted with a palladium catalyst or a nickel catalyst. By being contacted with a palladium catalyst, the vinyl groups of unreacted monomers remaining in the polymerization reaction product are saturated, and irritability and odor when added to the cosmetic material can be reduced. Examples of palladium catalysts include palladium compounds such as: tetrakis (triphenylphosphine)palladium (0) and dichlorobis (triphenylphosphine)palladium (II), and the like; and carbon-supported palladium, carbon-supported palladium hydroxide, platinum oxide, and the like, but the palladium catalyst is not necessarily limited thereto. A preferred catalyst is carbon-supported palladium. Other metals such as nickel and the like are also conceivable as catalysts, but because the polymerization reaction product contains an acidic group when nickel is used as the catalyst, the nickel is eluted into the reaction system a small amount at a time under acidic conditions, which is not preferable. On the other hand, this type of problem does not occur for the most part with palladium, which is a noble metal, and particularly with a carbon-supported palladium catalyst, which is a heterogeneous catalyst, and thus such catalysts can be suitably used as the catalyst of the present invention.

The temperature when contacting the polymerization reaction product with the palladium catalyst is 50 to 200°C, and preferably 70 to 130°C. The pressure is 1 to 1000 kg/cm$^2$ (absolute pressure), and preferably 2 to 100 kg/cm$^2$. The contact time is 1 to 15 hours, and preferably 3 to 10 hours. The reaction can be performed in a solvent, and the solvent that is used during polymerization may be used as is, or the solvent may be substituted. The solvents that can be used are the same as those described for the polymerization reaction.

Other steps such as stripping, re-precipitation, filtration, and the like may be performed between the first and second steps. In addition, a step such as stripping, re-precipitation, filtration, pulverization, classification, or the like can be performed after the second step. In addition, the second step may be omitted, and a step such as stripping, re-precipitation, filtration, pulverization, classification, or the like can be performed after the first step.

The presence or absence of unreacted monomers in the copolymer obtained as described above can be confirmed by the peak integrated value (5.5 to 6.5 ppm) of ethylenic unsaturated groups through $^1$H-NMR, and the end point of the reaction can be confirmed by the disappearance or reduction of the peak derived from the ethylenic unsaturated groups. For example, the ratio of the peak integrated value of ethylenic unsaturated groups to the product of the integrated value of methyl groups derived from the radical polymerizable monomer (0 to 0.3 ppm) and the mass percent of radical polymerizable monomer when prepared (ratio of residual unsaturation) can be compared. The ratio of residual unsaturation in the copolymer is 0.1 or less, and preferably 0.02 or less.

The copolymer included in the radical copolymer composition of the present invention can be added to a cosmetic material as is or in the form of a composition dissolved in a solvent, dispersed in a dispersing medium, or the like.

[Polymerization Inhibitor]

The radical copolymer composition of the present invention contains the polymerization inhibitor at a concentration where the total concentration of the polymerization inhibitor relative to the total mass of the radical copolymer and the polymerization inhibitor is 50 ppm by mass or less. Preferably, the total concentration of the polymerization inhibitor relative to the total mass of the radical polymerizable monomer and polymerization inhibitor is 30 ppm by mass or less, more preferably 10 ppm by mass or less, and even more preferably 5 ppm by mass or less.

The polymerization inhibitor includes one or more types selected from hindered phenol-based polymerization inhibitors, hydroquinone-based polymerization inhibitors, and catechol-based polymerization inhibitors. Examples of hindered phenol-based polymerization inhibitors include dibutylhydroxytoluene, 2,6-di-tert-butylphenol, 2,4-di-tert-butylphenol, 2-tert-butyl-4,6-dimethylphenol, 2,6-di-tert-butyl-4-methylphenol, and 2,4,6-tri-tert-butylphenol. Examples of hydroquinone-based polymerization inhibitors include methylhydroquinone, ethylhydroquinone, propylhydroquinone, tert-butylhydroquinone, 2,5-di-tert- butylhydroquinone, hydroquinone monomethyl ether, p-benzoquinone, and 2,5-diphenylparabenzoquinone. Examples of catechol-based polymerization inhibitors include catechol, 2-methylcatechol, 3-methylcatechol, 4-methylcatechol, 2-ethylcatechol, 3 ethylcatechol, 4-ethylcatechol, 2-propylcatechol, 3-propylcatechol, 4-propylcatechol, 2-n-butylcatechol, 3-n-butylcatechol, 4-n-butylcatechol, 2-tert butylcatechol, 3-tert-butylcatechol, 4-tert-butylcatechol, and 3,5-di-tert-butylcatechol. In particular, the monomer composition of the present invention includes one or more type selected from dibutylhydroxytoluene (BHT), methyl hydroquinone (MEHQ), and 4-tert-butylcatechol

(TBC). These hindered phenol-based phenol polymerization inhibitors, hydroquinone-based polymerization inhibitors, and catechol-based polymerization inhibitors have been reported to be suspected to be mutagenic in some cases, and it is desirable to reduce the amount in order to reduce the potential risk.

[Method of Manufacturing Radical Copolymer Composition]

Furthermore, in one embodiment, the present invention also relates to a method of manufacturing the radical copolymer composition of the present invention, including: a step of bringing a liquid containing a radical polymerizable monomer into contact with an adsorbing agent selected from a group consisting of activated carbon and alumina. Generally, a crude monomer composition that is obtained is purified by distillation, or the like. In such cases, unintended polymerization and gelation may occur, and the quality of the monomer composition may be problematic. Therefore, the polymerization inhibitors are used without sufficiently reducing the concentration.

The activated carbon used in the manufacturing method of the radical copolymer composition of the present invention is not particularly limited, and is manufactured by activating carbon substances by reacting with a gas or chemical substance at high temperatures. The adsorbing agent can be used alone or in combination with adsorbing agents other than alumina and activated carbon, such as zeolite, silica, aluminum silicate, and the like. Activated carbon is preferably used as the adsorbing agent.

Contact between the adsorbing agent and the liquid containing the radical polymerizable monomer can be made without solvent or in the presence of a solvent. The temperature and pressure at which the step of contacting the adsorbing agent with the liquid containing the radical polymerizable monomer is performed are not particularly limited, but can be performed at 0°C to 200°C, preferably 5°C to 150°C, more preferably 10°C to 100°C, at atmospheric pressure.

The method of bringing the adsorbing agent into contact with the liquid containing the radical polymerizable monomer is not particularly limited, but can be performed by a fixed bed system in which the adsorbing agent is filled in a container and the liquid containing the radical polymerizable monomer is continuously supplied, a batch system in which stirring and mixing and solid-liquid separation are each performed in batch operations, a mobile layer system in which the adsorbing agent is a mobile layer and passed through the liquid containing the radical polymerizable monomer, or a fluidized bed system, in which a solid layer of adsorbing agent is fluidized by the liquid containing the radical polymerizable monomer. The step of bringing the adsorbing agent into contact with the liquid containing the radical polymerizable monomer is particularly preferably performed in a fixed bed system, and continuously supplying the liquid containing the radical polymerizable monomer to the fixed bed of activated carbon is more preferable.

In the case of the fixed bed system, the liquid containing the radical polymerizable monomer can be passed through the fixed bed of adsorbing agent only once, or the liquid containing the radical polymerizable monomer can be circulated by supplying the outlet liquid from the fixed bed of adsorbing agent back into the inlet. The circulating system is preferred because this system makes it easier to ensure sufficient contact time between the liquid and the adsorbing agent.

In a fixed bed system, the feed flow rate of the liquid containing the radical polymerizable monomer can be appropriately determined according to the type and concentration of the polymerization inhibitor and adsorbing agent. The feed flow rate of the liquid containing the radical polymerizable monomer is not particularly limited, but can be calculated as the feed flow rate of the liquid divided by the mass of the adsorbing agent (g-AD), and the flow rate can be 0.1 g/(min·g-AD) to 100 g/(min·g-AD), preferably 0.5 g/(min·g-AD) to 50 g/(min·g-AD), and more preferably 1.0 g/(min·g-AD) to 10 g/(min.g-AD). The feed flow rate is preferably within an appropriate range to ensure sufficient contact time with the adsorbing agent and proper removal of the polymerization inhibitor.

In a fixed bed system, if the liquid containing the radical polymerizable monomer is circulated, the circulation frequency can also be appropriately determined according to the type and concentration of the polymerization inhibitor and adsorbing agent. The circulation frequency is not particularly limited, but can be 0.1 to 20 times/hour, preferably 0.5 to 15 times/hour, and more preferably 1 to 10 times/hour. By setting the circulation frequency within the range above, the concentration of the polymerization inhibitor in the liquid becomes more uniform, which is preferable for proper removal of the polymerization inhibitor.

In a batch system, the stirring and mixing time of the liquid containing the radical polymerizable monomer can be appropriately determined according to the type and concentration of the polymerization inhibitor and adsorbing agent. The time for stirring and mixing the liquid containing the radical polymerizable monomer is not particularly limited, but can be 0.5 to 30 hours, preferably 1 to 20 hours, and more preferably 3 to 10 hours.

The manufacturing method for the radical copolymer composition of the present invention may include a step of bringing the adsorbing agent into contact with the liquid containing the radical polymerizable monomer and then adding a polymerization inhibitor that does not have a risk of mutagenicity, or the like, preferably a polymerization inhibitor other than BHT, MEHQ and TBC, such tetra(di-t-butyl hydroxyhydrosilicate) pentaerythryl and other silicic acid derivatives, alpha-tocopherol, propyl gallate, and the like.

[Cosmetic Material]

In one embodiment, the radical copolymer composition of the present invention can be used as a cosmetic raw material or a cosmetic material. When the radical copolymer of the present invention is added to a cosmetic material, the resulting form can be a solution with the copolymer dissolved in a solvent, a dispersion liquid with the copolymer dispersed in a dispersion medium, or a solid such as a powder, granules, blocks, or the like. The added amount of the radical copolymer in the cosmetic raw material or cosmetic material of the present invention is not particularly limited, and the cosmetic material of the present invention may contain, for example, 0.1 to 90 mass% of the radical copolymer, based on the total mass of the cosmetic raw material or cosmetic material. Specifically, the added amount of the radical copolymer can be changed appropriately within the range from 0.1 to 50 mass%, from 0.5 to 20 mass%, or 1 to 10 mass%, for example, depending on the type of the cosmetic material or cosmetic raw material.

Cosmetic raw materials and cosmetic materials of the present invention may be compositions containing at least one type selected from a group consisting of: (A) oil agents, (B) alcohols, (C) surfactants, (D) powders or colorants, (E) gelling agents or thickeners, (F) organically modified clay minerals, (G) silicone resins, (H) silicone gums, (I) silicone elastomers, (J) organically modified silicones, (K) ultraviolet light blocking component, and (L) water-soluble polymers.

(A) Oil Agents

Examples of the oil agents include animal oils, plant oils, synthetic oils, and the like, that are generally used in cosmetic materials. The oil agent may be a solid, semi-solid, or liquid, and may be non-volatile, semi-volatile, or volatile. The oil agent is used to impart lubricity to the skin or hair, and to soften the skin and impart a moisturized feel. The oil agent can also be used to dilute the copolymer of the present invention to obtain a copolymer composition, and in particular, is a liquid at 5 to 100°C. The oil is preferably at least one type selected from silicone-based oil agents (A1) and organic oil agents (A2), and the type and viscosity, or the like of these oils can be appropriately selected according to the type of cosmetic material and the application. These oil agents are added to the cosmetic raw material or cosmetic material of the present invention at the same time as the monomer composition.

(A1) Silicone-Based Oil Agent

Silicone-based oil agents are generally hydrophobic, and their molecular structure may be linear, cyclic, or branched. The viscosity of the silicone-based oil agent at 25°C is usually in a range of 0.65 to 100,000 $mm^2/s$, and preferably in a range of 0.65 to 10,000 $mm^2/s$. Furthermore, the silicone-based oil agent may and preferably exhibits volatility.

Specific examples of silicone-based oil agents include cyclic organopolysiloxanes, linear organopolysiloxanes, and branched organopolysiloxanes. Of these, linear organopolysiloxanes, branched organopolysiloxanes, and cyclic organopolysiloxanes that are volatile are preferable.

If the cosmetic material or cosmetic raw material of the present invention contains at least one type of silicone-based oil agent, the stability over time will be improved, and a refreshing tactile sensation that is unique to silicone oil can be achieved. Particularly preferably, of the silicone-based oil agents described above, decamethylcyclopentasiloxane, 1,1,1,3,5,5,5-heptamethyl-3-octyltrisiloxane (also referred to as "caprylyl methicone"), which is a linear organopolysiloxane having a viscosity in a low viscosity range of 2 to 6 mPa·s, tristrimethylsiloxymethylsilane (also referred to as "M3T"), and the like are used.

(A2) organic Oil Agent

The organic oil agent is exemplified by (A2-1) hydrocarbon oils, (A2-2) fatty acid ester oils, higher alcohols, higher fatty acids, oils and fats, and fluorine-based oil agents, and in the present invention, the organic oil agent is not particularly limited, but is preferably a liquid at temperatures of 5 to 100°C. Furthermore, hydrocarbon oils and/or fatty acid ester oils are preferred. These may be used alone or in combination, and can be used in combination with the silicone-based oil agent. By combining appropriate oil agents, the stability over time of the composition and/or cosmetic material can be improved, and a tactile sensation required for each cosmetic material can be imparted. A refreshing tactile sensation that is unique to silicone oil can be imparted by adding in the silicone-based oil agent, and a tactile sensation that is refreshing on the skin can be imparted by using an oil agent with high volatility. Furthermore, a moisturized sensation (also referred to as a "moisturized feel") and a smooth tactile sensation like that of damp skin or hair can be imparted by using a hydrocarbon oil and/or a fatty acid ester oil in combination with the silicone-based oil agent.

In addition to the above, oils and fats, higher alcohols, higher fatty acids, fluorine-based oils, and the like may be used as oil agents, and two or more of these may be used in combination. For example, two or more types of oil agents represented below may be used in combination. More specific examples of other oil agents that can be used in

the present invention are provided below. More specifically, use of one or more types selected from oils and fats, higher alcohols, higher fatty acids, and fluorine-based oil agents is exemplified.

(B) Alcohols

As the alcohol, one or two or more polyhydric alcohols and/or lower monohydric alcohols can be used. Of these, ethanol, 1,3-propanediol, 1,3-butylene glycol, sorbitol, dipropylene glycol, glycerin, and polyethylene glycol are particularly preferable.

The radical copolymer composition of the present invention or the cosmetic material or the cosmetic raw material containing the composition may contain surfactant (C) as an optional component. The surfactant (C) can be one or two or more types of surfactants, used in combination according to the objective, selected from a group consisting of (C1) silicone-based surfactants, (C2) anionic surfactants, (C3) cationic surfactants, (C4) nonionic surfactants, (C5) amphoteric surfactants, and (C6) semipolar surfactants.

The amount of the surfactant (C) that is added to the radical copolymer composition of the present invention or the cosmetic material or the cosmetic raw material containing the composition is not particularly limited. However, in order to stabilize the emulsion or dispersion, the surfactant (F) can be added to the emulsion composition or dispersion composition in a range of 0.05 to 90 mass%, preferably 0.1 to 50 mass%, and even more preferably 0.5 to 25 mass%, based on the weight of the composition.

(D) Powder or Colorant

The radical copolymer composition of the present invention or the cosmetic material or the cosmetic raw material containing the composition can further contain powders or colorants, particularly arbitrary powders used in cosmetic products (including powders and pigments used as colorants). Any powder or colorant can be used as long as the powder or colorant is one that is used in ordinary cosmetic materials, regardless of the shape (such as spherical, a rod-shape, needle-shape, plate-shape, sheet-shape, indefinite shape, spindle-shape, bowl-shape, raspberry-shape, and the like), the particle size (such as in an aerosol form, microparticle form, pigment class, and the like), and the particle structure (such as porous, non-porous, secondary aggregation, and the like) of the powder or colorant, and when these powders and/or colorants are added as a pigment, one or two or more types selected from inorganic pigment powders, organic pigment powders, and resin powders having an average particle diameter in the range of 1 nm to 20 $\mu$m are preferably added.

Specific examples of the powder or colorant include inorganic powders, organic powders, surfactant metal salt powders (metal soaps), colored pigments, pearl pigments, metal powder pigments, silicone elastomer powders, and the like. Composites of these can also be used. Note that these powders or colorants include those which function as ultraviolet light blocking components.

Furthermore, it is particularly preferable that some or all of these powders or colorants are subjected to a water-repellent treatment by commonly known means. Such a treatment allows for stable blending into the oil phase. Furthermore, these powders or colorants may be combined together, and powders and colorants that have been subjected to a surface treatment using a general oil agent, silicone compounds other than the organopolysiloxane copolymer of the present invention, or fluorine compounds or surfactants, or the like can also be used.

The aforementioned powder or colorant is preferably treated using another powder dispersant or surface treatment agent, and in particular, the powder or colorant may be dispersed or surface treated by a novel powder treating agent and treatment method proposed in International Patent Publication WO 2009/022621, Japanese Unexamined Patent Application 2011-148784, Japanese Unexamined Patent Application 2011-149017, Japanese Unexamined Patent Application 2011-246704, Japanese Unexamined Patent Application 2011-246705, Japanese Unexamined Patent Application 2011-246706, International Patent Publication WO 2009/022621, International Patent Publication WO 2011/049246, International Patent Publication WO 2011/049248, Japanese Patent Application 2011-286973, and the like, or the powder or colorant may be treated with a new powder treatment agent and the oil agent to form a slurry. These novel treatment agents excel even further in performance areas such as dispersion stability and the like and the effect of improving the inherent tactile sensation; therefore, when these novel treatment agents are used in combination with the radical copolymer composition of the present invention, an effect of further improving aspects such as the function, tactile sensation, storage stability, and the like of the cosmetic material is anticipated.

Furthermore, some or all of these powders or colorants can be subjected to a hydrophilizing treatment. Thereby, the powder or colorant according to an aqueous phase can be added.

Furthermore, some or all of these powders or colorants can be subjected to a hydrophobizing treatment and/or hydrophilizing treatment. Through such treatments, emulsification properties can be imparted to the powder itself. Examples of commercially available products include MZY-500SHE available from Tayca Co., Ltd., and the like.

One type or two or more types of the powder or colorant (D) may be used, if necessary, in the radical copolymer

composition of the present invention or the cosmetic material or the cosmetic raw material containing the composition, and the added amount is not particularly limited. Furthermore, the amount of the powder or colorant can be added within a range of 0.1 to 99.5 mass%, and preferably 1 to 99 mass%, of the total composition or cosmetic material. In particular, the added amount for a powdered solid cosmetic material is preferably within the range of 80 to 99 mass% of the entire cosmetic material.

(E) Gelling Agent or Thickener

The gelling agent is preferably an oil-soluble gelling agent, and specific examples include metal soaps such as aluminum stearate, magnesium stearate, zinc myristate, and the like; amino acid derivatives such as N-lauroyl-L-glutamic acid, $\alpha,\gamma$-di-n-butylamine, and the like; dextrin fatty acid esters such as dextrin palmitate, dextrin stearate, dextrin 2-ethylhexanoic acid palmitate, and the like; sucrose fatty acid esters such as sucrose palmitate, sucrose stearate, and the like; and benzylidene derivatives of sorbitol such as monobenzylidene sorbitol, dibenzylidene sorbitol, and the like. One or two or more of these can be used as necessary.

(F) Organically Modified Clay Minerals

Examples of the organically modified clay minerals include dimethylbenzyldodecylammonium montmorillonite clay, dimethyldioctadecylammonium montmorinite clay, dimethylalkylammonium hectorite, benzyldimethyl stearylammonium hectorite, distearyldimethylammonium aluminum magnesium chloride-treated magnesium aluminum silicate, and the like. Commercially available products thereof include Benton 27 (hectorite treated with benzyl dimethyl stearyl ammonium chloride, available from National Lead Co.), Benton 38 (hectorite treated with distearyl dimethyl ammonium chloride, available from National Lead Co.), and the like.

(G) Silicone Resin

The silicone resin is an organopolysiloxane having a highly branched structure, a net-like structure, or a cage-like structure, and is in a liquid state or a solid state at ambient temperature, and may be any silicone resin commonly used in cosmetic materials as long as the silicone resin does not contradict the object of the present invention. Examples of solid silicone resins include MQ resin, MDQ resin, MTQ resin, MDTQ resin, TD resin, TQ resin, and TDQ resin made from optional combinations of triorganosiloxy units (M unit) (organo groups are methyl groups only, or are methyl groups and vinyl groups or phenyl groups), diorganosiloxy units (D unit) (organo groups are methyl groups only, or are methyl groups and vinyl groups or phenyl groups), monoorganosiloxy units (T unit) (organo groups are methyl groups, vinyl groups, or phenyl groups), and siloxy units (Q unit). Furthermore, examples include trimethyl siloxysilicate, polyalkyl siloxysilicate, trimethyl siloxysilicate containing dimethylsiloxy units, and alkyl (perfluoroalkyl) siloxysilicate. It is particularly preferable that these silicone resins are oil-soluble and can be dissolved in (A).

The silicone resin forms a uniform film when applied to skin, hair, or the like, and provides a protective effect against drying and low temperature. Furthermore, the silicone resin having these branched unit tightly adheres to the skin, hair, and the like, and can impart luster and a transparent feel to skin, hair, and the like.

(H) Silicone Gum

In the present invention, ultra-high viscosity organopolysiloxanes, referred to as silicone gums, having a viscosity of 1,000,000 $mm^2/s$ or higher can also be used as silicone oils. Silicone gum is a linear diorganopolysiloxane with an ultra-high degree of polymerization, and is also referred to as a raw silicone rubber or an organopolysiloxane gum. Silicone gums have a measurable degree of plasticity due to the high degree of polymerization, and are thereby distinguished from the silicone-based oil agents described above. Examples of such a silicone raw rubber include substituted or unsubstituted organopolysiloxanes having dialkylsiloxy units (D units) such as dimethylpolysiloxanes, methylphenylpolysiloxanes, aminopolysiloxanes, methylfluoroalkylpolysiloxanes, and the like or substances having finely crosslinked structures thereof. A representative example is a substance expressed by the general formula:

$$R^{10}(CH_3)_2SiO\{(CH_3)_2SiO\}_s\{(CH_3)R^{11}SiO\}_tSi(CH_3)_2R^{10}$$

(wherein $R^{11}$ is a group selected from a vinyl group, a phenyl group, an alkyl group with 6 to 20 carbon atoms, an aminoalkyl group with 3 to 15 carbon atoms, a perfluoroalkyl group with 3 to 15 carbon atoms, and a quaternary ammonium salt group-containing alkyl group with 3 to 15 carbon atoms, and the end group $R^{10}$ is a group selected from an alkyl group with 1 to 8 carbon atoms, a phenyl group, a vinyl group, an aminoalkyl group with 3 to 15 carbon atoms, a hydroxyl group, and an alkoxy group with 1 to 8 carbon atoms. Furthermore, s = 2000 to 6000, t = 0 to 1000, and s + t = 2000 to

6000). Of these, dimethylpolysiloxane raw rubbers having a degree of polymerization of 3000 to 20,000 are preferable. Furthermore, an amino-modified methylpolysiloxane raw rubber having a 3-aminopropyl group, an N-(2-aminoethyl) 3-aminopropyl group, or the like in a side chain or at an end of the molecule is preferable. Furthermore, in the present invention, one type or a combination of two or more types of silicone gums can be used as necessary. The silicone gum can be added to the radical copolymer composition of the present invention or the cosmetic material or the cosmetic raw material containing the composition, either as is, or as a liquid gum dispersion (oil dispersion of silicone gum) with the silicone gum dispersed in an oily silicone.

Silicone gums have an ultra-high degree of polymerization, and therefore form a protective film having excellent residual properties on skin and hair and excellent air permeability. Thus, silicone gum is a component that can impart luster and gloss to particularly skin and hair, and can impart tension and a resilient texture to the entire skin and hair both during and after use.

The added amount of the silicone gum is, for example, in a range of 0.05 to 30 mass%, and preferably 1 to 15 mass%, based on the entire cosmetic material. Note that the silicone gum is easy to add if used as an emulsion composition prepared in a pre-emulsification step (also including emulsion polymerization), and can be stably added to the radical copolymer composition of the present invention or the cosmetic material or the cosmetic raw material containing the composition. If the added amount of the silicone gum is less than the abovementioned lower limit, the effect of imparting gloss to the skin and hair may be insufficient.

(I) Silicone Elastomer

The silicone elastomer can be added to the cosmetic material or cosmetic raw material in an arbitrary form in accordance with the purpose thereof, but in particular, in addition to the silicone elastomer powder described in the aforementioned "(D) Powder", adding the silicone elastomer as a crosslinkable organopolysiloxane is preferable. The silicone elastomer powder can also be used in the form of an aqueous dispersion liquid in the radical copolymer composition of the present invention or the cosmetic material or the cosmetic raw material containing the composition. Examples of commercially available products of such an aqueous dispersion liquid include BY29-129 and PF-2001 PIF Emulsion available from Dow Toray Co., Ltd. and the like. By adding these silicone elastomer powders in the form of a water-based dispersion (= suspension), the feel of use of the radical copolymer composition of the present invention or the cosmetic material or the cosmetic raw material containing the composition can be further improved, and thus such silicone elastomer powders are extremely useful.

As the crosslinkable organopolysiloxane, a non-emulsifying organopolysiloxane having a structure in which an organopolysiloxane chain is three-dimensionally crosslinked by reaction with a crosslinkable component or the like, and not having a hydrophilic part such as a polyoxyalkylene unit is preferable. Such crosslinkable organopolysiloxanes can be used without limitation irrespective of physical forms such as dilutions and properties, and of the preparation method, and the like, and $\alpha,\omega$-diene crosslinked silicone elastomers described in US Patent No. 5,654,362 (commercially available products include DOWSIL 9040 Silicone Elastomer Blend, DOWSIL 9041 Silicone Elastomer Blend, DOWSIL 9045 Silicone Elastomer Blend, and DOWSIL 9046 Silicone Elastomer Blend, available from The Dow Chemical Company, USA) are particularly preferred. Crosslinkable organopolysiloxanes that are fluid at room temperature can also be suitably used, and examples include DOWSIL 3901 LIQUID SATIN BLEND (available from The Dow Chemical Company, USA) and the like.

(J) Organically Modified Silicone

The organically modified silicone is preferably lipophilic. Specific examples include, in addition to the above, amino-modified silicones, amino polyether-modified silicones, epoxy-modified silicones, carboxyl-modified silicones, amino acid-modified silicones, carbinol-modified silicones, acrylic-modified silicones, phenol-modified silicones, amidoalkyl-modified silicones, amino glycol-modified silicones, and alkoxy-modified silicones. The organically modified silicones may have, in addition to the polysiloxane bond as the main chain, an alkylene chain, an aminoalkylene chain or a polyether chain of an extent such that the compound is not hydrophilic, and an organically modified group may be present in one or both of a side chain and an end of the polysiloxane chain. If the monomer composition of the present invention, or a cosmetic raw material or cosmetic material containing the copolymer obtained from the composition is used as a hair cosmetic material, amino-modified silicones, carbinol-modified silicones, aminopolyether-modified silicones or aminoglycol-modified silicones can be suitably used, and general examples include amino-modified silicones having a 3-aminopropyl group, an N-(2-aminoethyl)3-aminopropyl group or the like.

Hereinafter, higher alkyl-modified silicones, alkyl-modified silicone resins, and polyamide-modified silicone resins that are particularly preferable as organically modified silicones are described. The higher alkyl-modified silicone is a component that is waxy at room temperature and is useful as a cosmetic raw material. Therefore, this component can be used in the radical copolymer composition of the present invention or the cosmetic material or the cosmetic raw

material containing the composition. Examples of such higher alkyl-modified silicone waxes include methyl long-chain alkyl polysiloxanes capped at both ends of the molecular chain with trimethylsiloxy groups, dimethyl polysiloxane/methyl long-chain alkyl siloxane copolymers capped at both ends of the molecular chain with trimethylsiloxy groups, long-chain alkyl-modified dimethyl polysiloxane capped at both ends of the molecular chain, and the like. Commercially available products thereof include AMS-C30 Cosmetic Wax and 2503 Cosmetic Wax (available from The Dow Chemical Company, USA), and the like.

In the radical copolymer composition of the present invention or the cosmetic material or the cosmetic raw material containing the composition, the higher alkyl-modified silicone wax preferably has a melting point of 60°C or higher from the perspective of cosmetic durability and high temperature stability.

The alkyl-modified silicone resin is a component that imparts sebum durability, moisture retention, and a smooth tactile sensation on the skin to a cosmetic material, and can be suitably used in a waxy form at room temperature. As the alkyl-modified silicone resin, for example, a silsesquioxane resin wax described in Japanese PCT Application 2007-532754 is preferable. Examples of commercially available products of alkyl-modified silicone resins include SW-8005 C30 RESIN WAX and the like (available from The Dow Chemical Company, USA).

Examples of polyamide-modified silicones include the siloxane-based polyamide compounds described in US Patent No. 5,981,680 (Japanese Unexamined Patent Application 2000-038450) and Japanese PCT Application 2001-512164, and commercially available products thereof include 2-8178 Gellant, 2-8179 Gellant, and the like (available from The Dow Chemical Company, USA). Such polyamide-modified silicones also function as thickeners/gelling agents for oily raw materials, particularly silicone oils.

(K) Ultraviolet Light Blocking Component

The ultraviolet light blocking component includes inorganic ultraviolet light blocking components and organic ultraviolet light blocking components. If the radical copolymer composition of the present invention or the cosmetic material or the cosmetic raw material containing the composition is for use in a sunscreen, at least one inorganic or organic component is preferably included, and particularly an organic ultraviolet light blocking component. The radical copolymer of the present invention excels in compatibility with poorly-soluble organic ultraviolet light blocking components, such as hexyl diethylaminohydroxybenzoyl benzoate known as "Uvinul A", bis-ethylhexyloxyphenol methoxyphenyl triazine known as "Tinosorb S", 2-ethylhexyl 2-cyano-3,3-diphenylprop-2-enoate known as "Octocrylene", and other cinnamic acid-based ultraviolet absorbers, and can improve the blending stability.

In the radical copolymer composition of the present invention or the cosmetic material or the cosmetic raw material containing the composition, the ultraviolet light blocking component which can be suitably used is at least one selected from a group consisting of microparticulate titanium oxide, microparticulate zinc oxide, 2-ethylhexyl paramethoxycinnamate, 4-tert-butyl-4'-methoxydibenzoylmethane, hexyl diethylamino hydroxybenzoylbenzoate, bis-ethylhexyloxyphenol methoxyphenyl triazine, 2-ethylhexyl 2-cyano-3,3-diphenylpropa-2-enoate, and other benzophenone-based ultraviolet absorbers. These ultraviolet light blocking components are generally used, are easily available, and have a high ultraviolet light blocking effect, and therefore can be suitably used. In particular, it is preferable to use a combination of an inorganic and an organic ultraviolet light blocking component, and it is more preferable to use a combination of an ultraviolet light blocking component corresponding to UV-A and an ultraviolet light blocking component corresponding to UV-B.

(L) Water-Soluble Polymer

On the other hand, the radical copolymer composition of the present invention or the cosmetic material or the cosmetic raw material containing the composition can be an aqueous or emulsion composition containing a large amount of water-soluble components, and a water-soluble polymer (L) may be, and is preferably, added in accordance with the dosage form thereof. One or two or more types of water-soluble polymers can be used as the water-soluble polymer, and examples include natural water-soluble polymers, semisynthetic water-soluble polymers, and synthetic water-soluble polymers. Examples of other cationic water-soluble polymers include, particularly as components that can be favorably added in hair cosmetic materials, quaternary nitrogen-modified polysaccharides (e.g., cationically modified cellulose, cationically modified hydroxyethyl cellulose, cationically modified guar gum, cationically modified locust bean gum, and cationically modified starch, and the like), dimethyl diallyl ammonium chloride derivatives (e.g., dimethyl diallyl ammonium chloride-acrylamide copolymers, and polydimethyl methylene piperidinium chloride, and the like), and vinylpyrrolidone derivatives (e.g., a vinylpyrrolidone-dimethylamino ethylmethacrylate copolymer salt, a vinylpyrrolidone-methacrylamide propyltrimethyl ammonium chloride copolymer, and a vinylpyrrolidone-methylvinyl imidazolium chloride copolymer, and the like).

Other components ordinarily used in cosmetic materials can be added to the radical copolymer composition of the present invention or the cosmetic material or the cosmetic raw material containing the composition, within a range

that does not hinder the effect of the present invention, and examples of other such components include: organic resins, moisturizing agents, antiseptic agents, antimicrobial agents, perfumes, salts, antioxidants, pH adjusting agents, chelating agents, refreshing agents, anti-inflammatory agents, skin beautifying components (skin lightening agents, cell activating agents, rough skin improving agents, circulation promoters, skin astringents, anti-seborrheic agents, and the like), vitamins, amino acids, nucleic acids, hormones, inclusion compounds, and the like. These specific examples are common with, but not limited to, those examples specifically disclosed in paragraphs [0100] to [0113], and the like of Japanese Unexamined Patent Application 2011-149017.

The radical copolymer composition of the present invention or the cosmetic material or the cosmetic raw material containing the composition can contain natural plant extract components, seaweed extract components, or herbal medicine components, in accordance with the purpose thereof. Two or more types of these components may be added. These specific examples are common with, but not limited to, those examples specifically disclosed in paragraph [0115] and the like of Japanese Unexamined Patent Application 2011-149017.

Depending on the purposes thereof, the radical copolymer composition of the present invention or the cosmetic material or the cosmetic raw material containing the composition may contain a solvent such as light isoparaffin, ether, LPG, N-methylpyrrolidone, next generation CFCs, and the like, in addition to water such as purified water, mineral water, and the like.

Furthermore, at least one type of component selected from a group consisting of acrylic silicone dendrimer copolymers and alkyl-modified silicone resin waxes may be used in the radical copolymer composition of the present invention or the cosmetic material or the cosmetic raw material containing the composition. These components are film-forming components similar to the copolymer included in the radical copolymer composition of the present invention, but these components are not components having washability. Therefore, these components are preferably added within a range that does not impair the technical effects of the present invention.

As acrylic silicone dendrimer copolymers, for example, vinyl-based polymers having a carbosiloxane dendrimer structure at a side chain as described in Japanese Patent No. 4009382 (Japanese Unexamined Patent Application 2000-063225) are particularly preferred. Examples of commercially available products include FA 4001 CM Silicone Acrylate and FA 4002 ID Silicone Acrylate available from Dow Toray Co., Ltd. and the like.

As the alkyl-modified silicone resin wax, for example, silsesquioxane resin wax described in Japanese PCT Application 2007-532754 is preferable.

The radical copolymer composition of the present invention or the cosmetic material or the cosmetic raw material containing the composition may be in any form, such as liquid, emulsion, cream, solid, paste, gel, powder, multilayer, mousse, or spray.

The copolymer included in the radical copolymer composition of the present invention can form a film that excels in washing performance while also having water resistance and sebum resistance, on the skin or hair, and thus a cosmetic material that provides these functional films can be designed.

Specific products containing the radical copolymer composition of the present invention or the cosmetic material or the cosmetic raw material containing the composition include skin cleansing products, skin care products, make-up products, antiperspirant products, ultraviolet light blocking products, and other skin cosmetic products; hair washing agent products, hair dressing products, hair coloring products, hair grower products, hair-rinse products, hair conditioner products, hair treatment products, and other hair cosmetic products; bath cosmetic products; hair growth agents, hair tonics, analgesics, fungicides, anti-inflammatory agents, refreshing agents, and skin aging inhibitors, but there is no limitation there to these. The radical copolymer composition of the present invention comes into direct contact with the human body, particularly when used as a cosmetic product for skin or hair. Therefore, components with health risks, such as polymerization inhibitors that may be mutagenic, or the like were required to be removed as much as possible. The radical copolymer composition of the present invention satisfies these requirements, and the amount of polymerization inhibitor has been greatly reduced compared to conventional products.

## EXAMPLES

The present invention will be described in detail below based on examples, but the present invention is not limited to the following examples.

[Measuring Concentration of Polymerization Inhibitors]

A 0.25 g sample was diluted 20-fold in tetrahydrofuran (FUJIFILM Wako Pure Chemicals Corporation, for high-performance liquid chromatography, no stabilizer included), and 20 μL injected into a high-performance liquid chromatography analyzer (SHIMAZU Prominence-iLC-2030C3D) equipped with a reverse-phase column (Waters, Atlantis T3-3 μm (3.0 × 100 mm)). The temperature of the column oven was set to 40°C. A mixed solvent of water/methanol/tetrahydrofuran was used as the mobile phase, and the flow rate was 0.45 mL/min. The BHT concentration in the sample was

calculated using a calibration curve prepared from the peak area values of a standard sample of known concentration, the peak area value of the measured sample, and the dilution factor. The concentration of BHT with regard to the total mass of copolymer and BHT excluding solvent was calculated by dividing the BHT concentration in the sample by the concentration of non-volatile content in the sample. The BHT concentration described in the examples and comparative examples refers to the BHT concentration based on the total mass of copolymers and BHT.

[Measurement of Non-Volatile Content Concentration]

A 1.0 g sample was weighed in an aluminum dish and placed in an oven heated to 150°C. After 3 hours, the sample was removed and the weight of the remaining material was measured. The non-volatile content concentration was calculated from the following formula.

$$\text{Non-volatile content concentration} = \text{sample weight after heating} / \text{initial sample weight}$$

[Manufacture of Radical Polymerizable Monomer]

Radical polymerizable monomers having the following structures used in the examples and comparative examples were manufactured by the following method. Note that "Me" in the drawings refers to a methyl group.

[Formula 9]

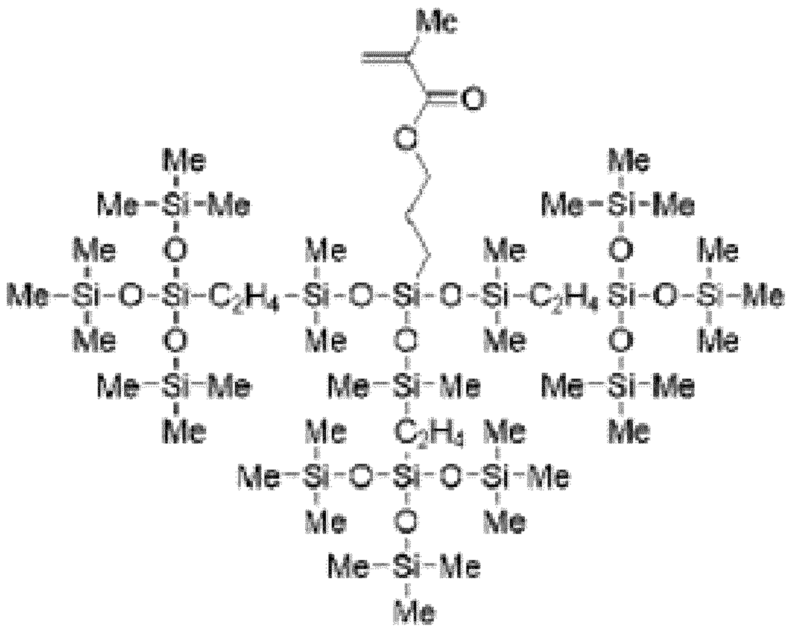

688 g of 1,1,1,5,5,5-hexamethyl-3-[(trimethylsilyl)oxy]-3-vinyltrisiloxane and 0.12 g of a toluene solution containing 5 mass% of 1,3-diethenyl-1,1,3,3-tetramethyldisiloxane platinum complex (hereinafter, referred to as platinum catalyst) were placed in a flask. The liquid temperature was heated to 70°C and bubbled with nitrogen gas containing 2 volume% of oxygen. 136 g of methacryloxypropyl tris(dimethylsiloxy)silane solution containing 500 ppm by mass of BHT was added by drops over 2 hours while maintaining the liquid temperature at 70°C. The liquid temperature was maintained at 70°C for 2 hours and then at 120°C for 2 hours. Next, after performing a single distillation at 120°C and 1 kPa for 5 hours, the liquid in the flask was collected. The BHT concentration in the recovered product was measured to be 160 ppm by mass.

[Method of Manufacturing Monomer Composition]

The device described in FIG. 1 was used as the separation device for the polymerization inhibitor used in Preparation Example 1.

A first end 17 of a first resin tube was placed in a beaker 18 containing a magnetic stirrer, and a second end 16 was connected to a suction port of a diaphragm pump 14. A first end 15 of a second resin tube was connected to a discharge port of the diaphragm pump, and a second end 13 was connected to an inlet of an activated carbon immobilized filter 11 (SC050X AKJ from Osaka Gas Chemicals Co., Ltd.: activated carbon loading capacity: 1.45 g). A first end 12 of a third resin tube was connected to an outlet of the filter, and a second end 19 was placed above the beaker 18.

[Preparation Example 1]

145 g of the radical polymerizable monomer manufactured by the aforementioned method was placed in a beaker and stirred at room temperature using a magnetic stirrer in the device depicted in FIG. 1. The ratio of activated carbon mass to liquid mass was 0.005. The liquid in the beaker was supplied to the filter at a flow rate of 11.0 g/min (7.59 g/(min·g-AD)), and the discharged liquid was returned to the beaker and circulated. The circulation frequency, calculated as the circulation flow rate divided by the total amount of liquid, was 2.3 times/hour. After 8 hours of circulation, a 1 g sample was taken from the beaker and analyzed for BHT concentration. The BHT concentration was 31.1 ppm by mass.

[Comparative Preparation Example 1]

The radical polymerizable monomer was used as it was without separating the polymerization inhibitor. The BHT concentration was 160 ppm by mass.

[Comparative Preparation Example 2]

300 g of radical polymerizable monomer manufactured in accordance with the aforementioned method was placed in a flask, depressurized with a vacuum pump to 2.0 kPa, and then heated with a mantle heater until the liquid temperature reached 125°C. After 3 hours of single distillation under these conditions, a 1 g sample of the liquid in the flask was collected and the BHT concentration was measured. The BHT concentration was 145.2 ppm by mass.

[Comparative Preparation Example 3]

The same method as Comparative Preparation Example 2 was used, except that the duration of the single distillation was 6 hours. The BHT concentration was 139.2 ppm by mass.

[Comparative Preparation Example 4]

The same method as Comparative Preparation Example 2 was used, except that the duration of the single distillation was 9 hours. The BHT concentration was 134.2 ppm by mass.

[Example 1]

290 g of the radical polymerizable monomer obtained by the Preparation Example 1 with a BHT concentration of 31.1 ppm by mass was placed in a beaker and stirred at room temperature using a magnetic stirrer. The discharged liquid was circulated for 3 hours by feeding to a filter and returning the liquid to the beaker using a diaphragm pump. 74.2 g of the radical polymerizable monomer solution, 9.3 g of butyl acrylate (Fujifilm Wako Pure Chemicals Corporation) and 102.1 g of methyl methacrylate (Mitsubishi Chemical, Acryester M) were mixed to obtain a monomer mixture solution. In addition, 4.1 g of dimethyl 2,2'-azobis(isobutyric acid) (FUJIFILM Wako Pure Chemicals Corporation, V-601) was dissolved in 68 g of 2-propanol to obtain a catalyst solution. 175g of 2-propanol was added to a 1 L flask, the temperature was raised to 65°C, and then the monomer mixture solution and the catalyst solution obtained above were added by drops at a constant flow rate over 3 hours at the same temperature. Next, the mixture was heated to 75°C and aged at that temperature for 12 hours. 336 g of isododecane (Maruzen Oil Co., Ltd., Marcasol R) was added, and then the 2-propanol in the solution was distilled off at 20 kPa and 120°C until the concentration of non-volatile content was 40 mass%. A 1 g sample was taken and analyzed for BHT concentration. The BHT concentration was at or below the lower detection limit (5 ppm by mass), and BHT could not be detected.

[Comparative Example 1]

The same process as Example 1 was performed except that a radical polymerizable monomer having a BHT concentration of 160 ppm by mass obtained in Comparative Preparation Example 1 was used instead of the radical polymerizable monomer having a BHT concentration of 31.1 ppm by mass obtained in Preparation Example 1. The BHT concentration was 75.0 ppm by mass.

[Comparative Example 2]

The same process as Example 1 was performed except that a radical polymerizable monomer having a BHT concentration of 145.2 ppm by mass obtained in Comparative Preparation Example 2 was used instead of the radical polymerizable monomer having a BHT concentration of 31.1 ppm by mass obtained in Preparation Example 1. The BHT concentration was 63.0 ppm by mass.

[Comparative Example 3]

The same process as Example 1 was performed except that a radical polymerizable monomer having a BHT concentration of 139.2 ppm by mass obtained in Comparative Preparation Example 3 was used instead of the radical polymerizable monomer having a BHT concentration of 31.1 ppm by mass obtained in Preparation Example 1. The BHT concentration was 60.3 ppm by mass.

[Comparative Example 4]

The same process as Example 1 was performed except that a radical polymerizable monomer having a BHT concentration of 134.2 ppm by mass obtained in Comparative Preparation Example 4 was used instead of the radical polymerizable monomer having a BHT concentration of 31.1 ppm by mass obtained in Preparation Example 1. The BHT concentration was 58.3 ppm by mass.

Table 1 Test conditions and BHT concentrations for Example 1 and Comparative Examples 1 to 4

[Table 1]

| | Units | Example 1 | Comparative Example 1 | Comparative Example 2 | Comparative Example 3 | Comparative Example 4 |
|---|---|---|---|---|---|---|
| Adsorption treatment of monomer | - | Present | Absent | Absent (Distillation method) | Absent (Distillation method) | Absent (Distillation method) |
| BHT concentration in radical polymerizable monomer composition | ppm by mass | 31.1 | 160 | 145.2 | 139.2 | 134.2 |
| BHT concentration in radical copolymer composition | ppm by mass | At or below detection limit | 75.0 | 63.0 | 60.3 | 58.3 |

As can be seen from Table 1, which summarizes the results of the Example and Comparative Examples, it was found that the method of the present invention can provide a copolymer in which the total concentration of the polymerization inhibitor relative to the total mass of the radical polymerizable monomer and polymerization inhibitor is 50 ppm by mass or less. On the other hand, the single distillation method used in the Comparative Examples 2 to 4 resulted in BHT concentrations exceeding 50 ppm by mass, even after prolonged treatment.

**DESCRIPTION OF SYMBOLS**

11 Activated carbon immobilized filter
12 First end of third resin tube

13 Second end of second resin tube
14 Diaphragm pump
15 First end of second resin tube
16 Second end of first resin tube
17 First end of first resin tube
18 Beaker
19 Second end of third resin tube

## Claims

**1.** A radical copolymer composition, comprising a radical copolymer of:

a) a radical polymerizable monomer having a radical polymerizable organic group and an organic silicon-containing organic group in a molecule; and
b) a radical polymerizable monomer other than component a),

wherein the total concentration of a polymerization inhibitor based on the total mass of the radical copolymer and the polymerization inhibitor is 50 ppm by mass or less.

**2.** The radical copolymer composition according to claim 1, wherein the organic silicon-containing organic group in the radical polymerizable monomer of component a) is selected from a carbosiloxane dendrimer structure and a branched or linear siloxane structure.

**3.** The radical copolymer composition according to Claim 1 or Claim 2, wherein the radical polymerizable monomer of component a) is expressed by the following general formula (1):

[Formula 1]

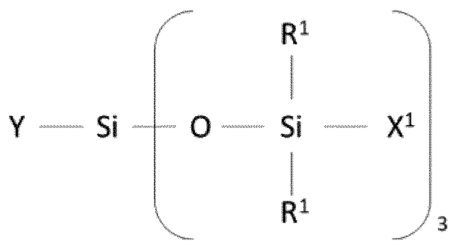

(1)

{where

Y is a radical polymerizable organic group,
$R^1$ represents an alkyl group, an aryl group or a trimethylsiloxy group;
$X^1$ represents a silylalkyl group expressed by the following general formula (2) when i = 1:

[Formula 2]

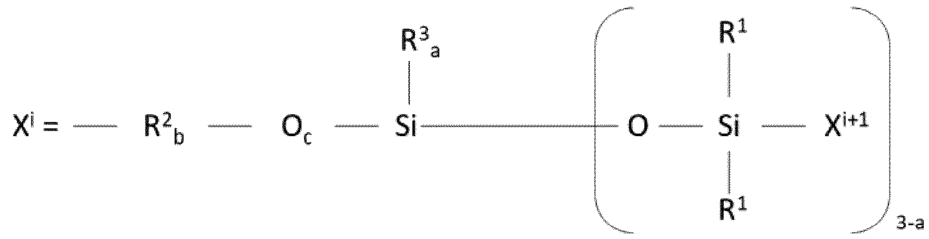

(2)

(where
$R^1$ represents the same groups as defined for general formula (1);
$R^2$ represents an alkylene group with 2 to 10 carbon atoms;
$R^3$ represents a group selected from a group consisting of alkoxy groups, hydroxyl groups, alkyl groups, aryl groups, and trimethylsiloxy groups;
$X^{i+1}$ represents a group selected from a group consisting of hydrogen atoms, alkyl groups with 1 to 10 carbon atoms, aryl groups, and the above silylalkyl groups, where i is an integer from 1 to 10, indicating the hierarchy of the silylalkyl group;
a is an integer from 0 to 3; and
band care 0 or 1.)}

**4.** The radical copolymer composition according to any one of claims 1 to 3, wherein the radical polymerizable organic group includes a (meth)acryloyl group.

**5.** The radical copolymer composition according to any one of claims 1 to 4, wherein the polymerization inhibitor includes one or more selected from hindered phenol-based polymerization inhibitors, hydroquinone-based polymerization inhibitors, and catechol-based polymerization inhibitors.

**6.** The radical copolymer composition according to any one of claims 1 to 5, wherein the polymerization inhibitor includes one or more selected from dibutylhydroxytoluene (BHT), hydroquinone monomethyl ether (MEHQ), and 4-tert-butylcatechol (TBC).

**7.** The radical copolymer composition according to any one of claims 1 to 7, that is a cosmetic raw material or a cosmetic material.

**8.** A method of manufacturing the radical copolymer composition according to any one of claims 1 to 8, comprising:
a step of bringing a liquid containing a radical polymerizable monomer into contact with an adsorbing agent selected from a group consisting of activated carbon and alumina.

**9.** The manufacturing method according to claim 9, comprising:
a step of continuously supplying a liquid containing the radical polymerizable monomer to a fixed bed of the adsorbing agent

**10.** The manufacturing method according to claim 10, further comprising:
a step of circulating the liquid containing the radical polymerizable monomer by resupplying the outlet liquid of the fixed bed of the adsorbing agent to the inlet.

**11.** The manufacturing method according to any one of claims 9 to 11, further comprising:
a step of adding a polymerization inhibitor other than BHT, MEHQ, and TBC after bringing the adsorbing agent into contact with the liquid containing the radical polymerizable monomer.

**12.** A cosmetic material or cosmetic raw material, comprising:
the radical copolymer composition according to any one of claims 1 to 8.

FIG. 1

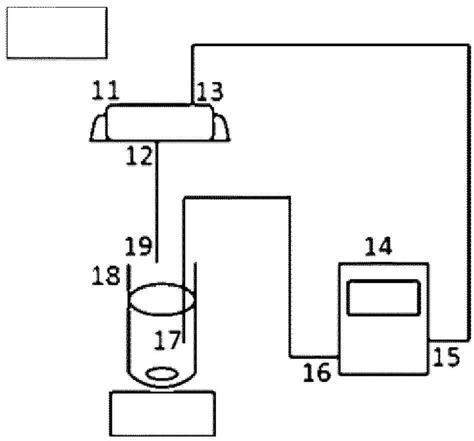

**INTERNATIONAL SEARCH REPORT**

International application No.
**PCT/JP2021/046062**

**A. CLASSIFICATION OF SUBJECT MATTER**

***C08F 220/10***(2006.01)i; ***C08F 230/08***(2006.01)i; ***C08K 5/13***(2006.01)i; ***C08L 101/10***(2006.01)i; ***A61K 8/00***(2006.01)i; ***A61K 8/89***(2006.01)i
FI: C08F230/08; A61K8/89; A61K8/00; C08L101/10; C08K5/13; C08F220/10

According to International Patent Classification (IPC) or to both national classification and IPC

**B. FIELDS SEARCHED**

Minimum documentation searched (classification system followed by classification symbols)

C08F220/10; C08F230/08; C08K5/13; C08L101/10; A61K8/00; A61K8/89

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| A | JP 5-025188 A (NIPPON OIL & FATS CO., LTD.) 02 February 1993 (1993-02-02) entire text | 1-12 |
| A | WO 2013/118766 A1 (FUJIFILM CORP.) 15 August 2013 (2013-08-15) entire text | 1-12 |
| A | JP 2005-239889 A (NITTO DENKO CORP.) 08 September 2005 (2005-09-08) entire text | 1-12 |
| A | JP 2004-115790 A (TORAY IND., INC.) 15 April 2004 (2004-04-15) entire text | 1-12 |
| A | JP 2006-169234 A (L'OREAL SA) 29 June 2006 (2006-06-29) entire text | 1-12 |
| A | JP 2020-145328 A (HITACHI INDUSTRIAL EQUIPMENT SYSTEMS CO., LTD.) 10 September 2020 (2020-09-10) entire text | 1-12 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **02 February 2022** | **15 February 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2021/046062**

| Patent document cited in search report | Publication date (day/month/year) | Patent family member(s) | Publication date (day/month/year) |
|---|---|---|---|
| JP 5-025188 A | 02 February 1993 | (Family: none) | |
| WO 2013/118766 A1 | 15 August 2013 | US 2014/0370250 A1<br>entire text<br>EP 2851402 A1 | |
| JP 2005-239889 A | 08 September 2005 | US 2005/0191456 A1<br>entire text<br>KR 10-2006-0042239 A<br>CN 1660952 A | |
| JP 2004-115790 A | 15 April 2004 | (Family: none) | |
| JP 2006-169234 A | 29 June 2006 | US 2006/0078523 A1<br>entire text<br>EP 1647262 A1 | |
| JP 2020-145328 A | 10 September 2020 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2014040512 A
- JP 2004115790 A
- JP 2006169234 A
- JP H111530 A
- WO 2009022621 A
- JP 2011148784 A
- JP 2011149017 A
- JP 2011246704 A
- JP 2011246705 A
- JP 2011246706 A
- WO 2011049246 A
- WO 2011049248 A
- JP 2011286973 A
- US 5654362 A
- JP 2007532754 W
- US 5981680 A
- JP 2000038450 A
- JP 2001512164 W
- JP 4009382 B
- JP 2000063225 A